# FASCICULE DE BREVET EUROPEEN

(11) **EP 1 162 937 B1**
(45) Date de publication et mention de la délivrance du brevet: **26.07.2006**
(21) Numéro de dépôt: 00912729.1
(22) Date de dépôt: 22.03.2000
(51) Int. Cl.: A61K 31/715, A61K 45/06

(54) **COMPOSITIONS A BASE D'UN SULFATE DE CHONDROITINE ET DE CHITOSAN POUR PREVENIR OU TRAITER LES AFFECTIONS RHUMATOLOGIQUES PAR VOIE GENERALE**
AUF CHONDROITINSULFAT UND CHITOSAN BASIERTE ZUSAMMENSETZUNGEN ZUR VERHÜTUNG ODER BEHANDLUNG RHEUMATISCHER ERKRANKUNGEN
COMPOSITIONS BASED ON CHONDROITIN SULPHATE AND CHITOSAN FOR PREVENTING OR TREATING RHEUMATIC DISORDERS BY GENERAL ADMINISTRATION

(30) Priorité: 22.03.1999 FR 9903538
(43) Date de publication de la demande: 19.12.2001
(73) Titulaire: VIRBAC, 06516 Carros (FR)
(72) Inventeur: Derrieu, Guy, 06800 Cagnes-sur-Mer (FR); Pougnas, Jean-Luc, 33500 Libourne (FR)
(74) Mandataire: Vialle-Presles, Marie José
(86) Numéro de dépôt international: PCT/FR2000/000721
(87) Numéro de publication internationale: WO 2000/056275

(56) Documents cités:
- EP-A- 0 454 599
- EP-A- 0 637 450
- EP-A- 0 941 735
- WO-A-94/00135
- WO-A-96/02259
- WO-A-98/22114
- WO-A-98/58011
- GB-A- 2 266 239
- GB-A- 2 286 528
- US-A- 5 145 841
- US-A- 5 166 187
- US-A- 5 364 845
- DATABASE WPI Week 199441 Derwent Publications Ltd., London, GB; AN 1994-329956 XP002144836 & JP 06 256221 A (KIBUN FOOD CHEMIPHAR KK) , 13 septembre 1994 (1994-09-13)

## Description

La présente invention a pour objet l'utilisation de préparations à base de sulfate de chondroïtine et de chitosan pour la réalisation de compositions destinées à prévenir ou à traiter les affections rhumatologiques et, en particulier, les arthropathies dégénératives, par voie générale.

Les articulations et les différents tissus conjonctifs qui les constituent (cartilages, fibro-cartilages, membranes synoviales, ligaments) sont constamment soumis à des contraintes mécaniques et à des stress qui peuvent conduire à des pathologies inflammatoires comme les arthrites ou dégénératives comme les arthroses, qui sont responsables de leur blocage. Ces affections peuvent être aiguës au niveau des articulations du cou, des épaules, du dos, des hanches, des membres antérieurs telles que les coudes et les poignets, des membres postérieurs comme les genoux et les chevilles, ainsi que des doigts des mains ou des pieds. Ces pathologies sont très fréquentes et touchent aussi bien les hommes que les animaux.

On utilise en rhumatologie humaine et vétérinaire principalement deux familles de composés anti-inflammatoires : les glucocorticostéroïdes et les A.I.N.S. (ou Anti-Inflammatoires Non Stéroïdiens : salycilés, indoliques et apparentés, propioniques, pyrazolés, anthranyliques). Ces composés, bien que soulageant la douleur et réduisant l'état inflammatoire des tissus conjonctifs lors de leur utilisation, n'ont qu'une fonction palliative de la douleur et ne permettent pas un retour à l'état normal par la reconstitution des tissus. Aussi, ces traitements deviennent-ils à la longue inefficaces, voire nocifs, car ils peuvent bloquer les processus naturels de défense de l'organisme et conduire à une destruction des tissus conjonctifs.

Les tissus conjonctifs, lorsqu'ils sont soumis à des efforts, des stress et surtout lorsqu'ils sont le siège de lésions, produisent naturellement de fortes quantités de collagène et de protéoglycanes (PG), composants majeurs de ces tissus, afin de se reconstituer.

Ces composés, ainsi que leurs mécanismes de biosynthèse et d'action, sont parfaitement connus et décrits dans la littérature.

Le collagène est fabriqué à partir d'acides aminés, en particulier à partir de proline, de glycine et de lysine, et sa biosynthèse est stimulée par la présence de glucosamine.

Les protéoglycanes, qui sont de larges complexes macromoléculaires, sont, quant à eux, constitués par de longues chaînes de sucres modifiés dénommées glycosaminoglycanes (GAG) telles que l'acide hyaluronique, les sulfates de chondroïtine ou encore l'héparine, et dont la glucosamine est un précurseur.

Dans les processus de reconstitution des tissus conjonctifs, le collagène et les protéoglycanes forment une matrice qui confère aux tissus leurs propriétés mécaniques. Cependant, les processus de biosynthèse *in vivo* du collagène et des protéoglycanes à partir des molécules précurseurs sont relativement longs, ce qui constitue un frein à la réparation de ces tissus.

Aussi, pour favoriser la reconstitution des tissus conjonctifs, certains traitements consistent à amener dans l'organisme des molécules précurseurs exogènes.

C'est ainsi qu'il a été proposé, dans le Brevet Américain n° 5,145,841, de traiter les affections rhumatologiques de nature inflammatoire et, en particulier, les arthrites, en injectant, soit directement dans les tissus où règne l'inflammation, soit dans la circulation générale (par voie intramusculaire, sous-cutanée ou intraveineuse), des compositions comprenant au moins deux composés choisis parmi l'acide hyaluronique, les corticoïdes et les polysaccharides sulfatés du type sulfates d'héparine et de chondroïtine, ces derniers étant, de préférence, utilisés sous la forme de complexes avec un ion métallique.

Par ailleurs, les Brevets Américains n° 5,364,845 et n° 5,587,363 ainsi que la Demande de Brevet Européen n° 0 693 928 décrivent des compositions destinées à protéger et réparer les tissus conjonctifs, qui sont, elles, prévues pour être administrées par voie orale et qui sont constituées par des associations entre un aminosucre et un glycosaminoglycane avec éventuellement un sel de manganèse. L'aminosucre est choisi parmi la glucosamine, ses sels et les mélanges de ces derniers, tandis que le glycosaminoglycane est choisi parmi les sulfates de chondroïtine, leurs sels et leurs mélanges.

Bien que ces associations aient montré un effet synergique, comparativement à des compositions contenant soit de la glucosamine seule, soit un sulfate de chondroïtine seul, on sait que les molécules exogènes ainsi apportées à l'organisme et, en particulier, les sulfates de chondroïtine et leurs sels, sont dégradées de manière importante *in vivo*. Cette dégradation réduit la quantité de molécules précurseurs susceptibles d'être utilisée pour la reconstruction des tissus conjonctifs et, partant, le bénéfice thérapeutique escompté, notamment dans le cas où ces molécules sont administrées par voie orale.

Il est, de ce fait, souhaitable de disposer de compositions d'efficacité thérapeutique améliorée.

Le chitosan est un polysaccharide que l'on obtient par une N-désacétylation plus ou moins totale de la chitine. L'hydrolyse de la chitine, homopolymère de N-acétyl-D-glucosamine et du chitosan conduit à la formation de glucosamine, précurseur principal des sucres intervenant dans la synthèse des glycosaminoglycanes.

Le chitosan a des propriétés filmogènes, reconstituantes, antibactériennes, antifongiques, cicatrisantes reconnues, qui ont conduit à son utilisation en médecine, notamment en tant que constituant de biomatériaux destinés à être utilisés en chirurgie orthopédique, plastique ou reconstructrice, soit comme peau artificielle, soit comme matrice propre à permettre à des cellules osseuses, nerveuses ou cutanées, de se régénérer. Ces utilisations sont par exemple décrites dans le Brevet Américain n° 5,166,187 et dans la Demande Internationale n° WO 96/02259.

Récemment, DENUZIERE et *al. (Electrophoresis,* 1997, 18, 745-750) ont montré, *in vitro,* que le chitosan protégeait, à un pH physiologique, les sulfates de chondroïtine de l'hydrolyse, lorsque ces sulfates étaient au préalable complexés, en milieu aqueux, avec le chitosan.

Le chitosan, ses sels et ses dérivés sont définis plus en détail dans de nombreux ouvrages, en particulier dans l'ouvrage de MUZZARELLI intitulé *"The Polysaccharides"* (1985) Academic-Press.

Or, les Inventeurs ont constaté que l'association d'un sulfate de chondroïtine ou d'un sel de celui-ci avec, soit du chitosan, soit un sel ou un dérivé de ce dernier, soit encore un sel d'un tel dérivé, permet, de manière inattendue, d'obtenir des compositions qui présentent un rapport effet thérapeutique/dose de sulfate de chondroïtine notablement supérieur à celui présenté par les compositions proposées dans US-A-5,364,845, US-A-5,587,363 et EP-A-0 693 928.

Ainsi, pour des doses de sulfate de chondroïtine administrées équivalentes, voire inférieures, des compositions comprenant à la fois un sulfate de chondroïtine (ou un sel d'un tel sulfate) et du chitosan (ou un sel, un dérivé ou un sel d'un dérivé du chitosan) se sont révélées améliorer plus rapidement les signes cliniques que les compositions proposées dans l'état de la technique précité ; en particulier, les compositions préparées par les Inventeurs ont montré qu'elles permettaient de diminuer plus rapidement les signes de douleurs et d'améliorer plus vite la mobilité, ainsi que la tolérance à l'effort.

La présente invention se rapporte, donc, à l'utilisation d'une préparation comprenant :
a) de 1 à 50% en poids, par rapport au poids total de la préparation, d'au moins un composé choisi parmi les sulfates de chondroïtine et leurs sels, et
b) de 1 à 66% en poids, par rapport au poids total de la préparation, d'au moins un composé choisi parmi le chitosan, ses sels, ses dérivés et les sels de ces dérivés,
pour la réalisation d'une composition pour prévenir ou traiter les affections rhumatologiques par voie générale.

Au sens de la présente invention, on entend par *"voie générale",* toute voie d'administration de la composition permettant une distribution systémique des principes actifs qu'elle renferme, c'est-à-dire la voie entérale (orale et rectale), la voie parentérale (intramusculaire, intraveineuse et sous-cutanée) et la voie transdermique, à l'exclusion de toute application *in situ* de ladite composition.

Selon une première disposition préférée de l'invention, la préparation comprend un sulfate de chondroïtine sodique.

Selon une autre disposition préférée de l'invention, la préparation comprend un composé choisi parmi le chitosan, ses sels d'acide adipique, d'acide ascorbique, d'acide formique, d'acide glycolique et d'acide lactique, les N-acyl chitosans, les N-carboxyalkyl chitosans, les N-carboxyacyl chitosans, les O-carboxyalkyl chitosans, les déoxyglycit-1-yl chitosans, les hydroxyalkyl chitosans et leurs sels obtenus par addition avec des acides organiques ou inorganiques.

Préférentiellement, le chitosan est obtenu par une N-désacétylation d'au moins 80% de la chitine et est choisi parmi ceux proposés par les sociétés japonaises KOYO, référence SK-400F80 ou SK-50SEP avec une désacétylation supérieure à 85%, KATAKURA, référence CTA-1 ou CTA-2 avec une désacétylation supérieure à 90% pour la première et supérieure à 80% pour la seconde, YAIZU, référence LL ou LL-40 avec une désacétylation supérieure à 80%, KYOWA, référence HW avec une désacétylation supérieure à 85%.

De manière avantageuse, la préparation comprend, de plus, une quantité efficace d'un ou plusieurs inhibiteurs de radicaux libres qui participent aux processus de protection, de traitement et de reconstitution des tissus conjonctifs.

On peut citer, à titre d'exemples d'anti-radicaux libres susceptibles d'être utilisés dans la compositions, la vitamine E et ses dérivés, la vitamine C et ses dérivés (en particulier l'ascorbate de manganèse et/ou l'ascorbate de glucosamine), les bioflavonoïdes, la superoxyde dismutase et ses sels, et les extraits de plantes connus pour présenter des effets anti-radicalaires tels que l'huile essentielle de romarin.

De manière avantageuse, la préparation comprend, encore, tout élément facilitant ou participant aux biosynthèses ou aux processus de protection, de traitement et de reconstitution des tissus conjonctifs tels que les oligo-éléments, sous formes organiques ou inorganiques, (le manganèse, le cuivre, le fer, le zinc), les vitamines et les nutriments.

Conformément à l'invention, la préparation est susceptible d'être utilisée pour réaliser une composition pharmaceutique, auquel cas cette dernière est, de préférence, formulée pour être administrée par voie orale.

En variante, la préparation peut également être utilisée pour réaliser un complément alimentaire apte, par une prise quotidienne ou en cure en complément de l'alimentation, à prévenir ou à traiter les affections rhumatologiques.

La composition pharmaceutique ou le complément alimentaire ainsi réalisés sont susceptibles de se présenter sous les différentes formes adaptées à une administration orale : solide (comprimés, gélules, tablettes à croquer, granulés, poudres pour suspension buvable), pâteuse ou liquide (sirops, solutions ou suspensions buvables). Aussi, la préparation est-elle susceptible de comprendre, en outre, différents additifs (agents liants, agents diluants, agents lubrifiants, agents d'écoulement, agents colorants, agents de sapidité, solvants) qui sont choisis en fonction de la forme que l'on souhaite donner à la composition pharmaceutique ou au complément alimentaire.

La composition préparée selon l'invention, qu'elle soit pharmaceutique ou qu'il s'agisse d'un complément alimentaire, comprend, de préférence, des quantités de sulfate de chondroïtine et de chitosan permettant d'apporter, par kg de poids vif et par jour :
a) de 5 à 25 mg de sulfate de chondroïtine sodique, et
b) de 5 à 50 mg de chitosan,
et ce, en une ou plusieurs prises.

De manière particulièrement préférée, cette composition comprend des quantités de sulfate de chondroïtine et d'un chitosan ayant un degré de N-désacétylation au moins égal à 80%, permettant d'apporter, par kg de poids vif et par jour :
a) de 10 à 20 mg et, de préférence, environ 15 mg de sulfate de chondroïtine sodique, et
b) de 10 à 20 mg et, de préférence, environ 15 mg de chitosan, en une ou plusieurs prises.

Elle est susceptible d'être utilisée aussi bien chez les animaux que chez l'homme dans la prévention et le traitement de toutes les pathologies rhumatologiques, qu'elles soient d'origine inflammatoire, métabolique ou dégénérative, chroniques ou aiguës.

A titre d'exemples de telles pathologies, on peut citer les arthrites, la polyarthrite rhumatoïde, la spondylarthrite ankylosante (ou syndromes apparentés tels que le syndrome de Fiessinger-Leroy-Reiter, le rhumatisme psoriasique), les périarthrites scapulo-humorales, les tendinites, les bursites, les arthroses (arthrose cervicale, arthrose vertébrale, coxarthrose) et la goutte.

L'exemple qui suit est destiné à illustrer l'invention, sans pour autant en limiter la portée.

### EXEMPLE

### 1. Mode opératoire

3 groupes de 20 chiens sans restriction de race, d'âge, de sexe, choisis dans une gamme de poids allant de 5 à 35 kg et présentant une arthrose à manifestation chronique se traduisant par une boiterie d'un membre depuis au moins 1 mois, ont été traités et suivis par leur propriétaire et par des vétérinaires.

La présence d'au moins une des lésions d'arthrose, figurant sur la fiche clinique, sur une ou plusieurs articulations du membre concerné (y compris l'articulation de l'épaule ou de la hanche) est confirmée à partir d'un cliché radiologique datant de moins de trois mois ou effectué le jour de la sélection des animaux.

Tous les traitements anti-inflammatoires par voie orale ou injectable et tous les produits à visée chondroprotectrice, ont été arrêtés au moins 7 jours avant la sélection des animaux.

On prépare selon des techniques connues de l'homme du métier des comprimés pour chien de formulations A et C et on utilise des capsules de formulation B du commerce.

Les comprimés de formulation A correspondent à une composition selon l'invention associée avec des excipients convenables.

Les capsules de formulation B correspondent aux produits commerciaux américains réalisés selon la méthode décrite dans les Brevets US n° 5,364,845 et n° 5,587,363. B1 correspond aux capsules dénommées "Cosequin (Regular Strength)" et B2 correspond aux capsules "Cosequin (Double Strength)".

Les comprimés de formulation C correspondent à une composition qui comprend des quantités de sulfate de chondroïtine et de glucosamine identiques à celles présentes dans la formulation B1, mais qui se présente sous la forme de comprimés ayant été préparés comme les comprimés A.

Le contenu final des comprimés A et C et des capsules B1 et B2 est illustré dans le tableau 1 qui suit. Les quantités sont exprimées en pourcentages en poids du poids total de la composition.

**Tableau 1**

| FORMULATIONS | | A | B | | C |
|---|---|---|---|---|---|
| | | | B1 | B2 | |
| - Sulfate de chondroïtine sodique | | 22,73 | | | 18,185 |
| - Chitosan | | 22,73 | | | |
| - Appétent | | 10,0 | | | |
| - Excipient pour comprimés | | 44,54 | | | 55,54 |
| - Glucosamine HCl 99+% | | | 51,23 | 51,23 | 22,73 |
| - Sulfate de chondroïtine sodique 95% + | | | 40,985 | 40,985 | |
| mélange de glycosaminoglycanes 5% | | | | | |
| - Ascorbate de manganèse | | | 7,785 | 7,785 | 3,455 |
| | - soit en ascorbate | | 6,76 | 6,76 | 3 |
| | - soit en manganèse | | 1,025 | 1,025 | 0,455 |
| *Poids d'un comprimé* | | *660 mg* | | | *1100 mg* |
| *Poids dans une capsule* | | | *488 mg* | *976 mg* | |

### a) mode de traitement du premier groupe

Chaque animal du premier groupe est traité par la composition A conforme à l'invention et présentée sous forme de comprimés, à raison de 1 prise par jour, le nombre de comprimés étant de 1 à 3 comprimés ½ par prise en fonction du poids de l'animal, pendant 6 semaines.

Chaque animal reçoit environ 15 mg de sulfate de chondroïtine sodique (ACS) et 15 mg de chitosan (KATAKURA, référence CTA - 1) par kg de poids vif et par jour.

### b) mode de traitement du deuxième groupe

Chaque animal du deuxième groupe est traité par la composition B1 [*Cosequin (Regular Strength)* ou B2 [*Cosequin (Double Strength)*] à raison de :
- 2 capsules par jour de B1 pendant 6 semaines pour les chiens de poids corporel compris entre 4,5 et 11,35 kg,
- 2 capsules de B2 pendant 6 semaines pour les chiens de poids corporel compris entre 11,35 et 22,24 kg,
- 3 capsules de B2 pendant 6 semaines pour les chiens de poids corporel entre 22,7 et 45,4 kg.

Chaque animal reçoit ainsi environ 30 mg de sulfate de chondroïtine et 37,5 mg de glucosamine par kg de poids vif et par jour.

### c) mode de traitement du troisième groupe

Chaque animal du deuxième groupe est traité par la composition C à raison de :
- 2 comprimés pour les chiens de poids corporel compris entre 4,5 et 11,35 kg,
- 4 comprimés pour les chiens de poids corporel compris entre 11,35 et 22,24 kg,
- 6 comprimés pour les chiens de poids corporel compris entre 22,7 et 45,4 kg.

Chaque animal reçoit ainsi environ 30 mg de sulfate de chondroïtine et 37,5 mg de glucosamine par kg de poids vif et par jour.

### d) paramètres étudiés :

Le propriétaire de chaque animal étudie, en faisant référence au comportement de son animal avant le traitement :
- la facilité à se mouvoir de l'animal et la boiterie de l'animal lors de ses déplacements,
- la souffrance de l'animal et les plaintes spontanées ou quand il est caressé.

Le vétérinaire, pendant les visites à 30 et 60 jours après le début du traitement, étudie :
- les difficultés à se lever ou à gravir un obstacle,
- la tolérance à l'exercice, appréciée en interrogeant le propriétaire.
- la boiterie, appréciée par examen clinique,
- la douleur, appréciée par palpation-pression et par mobilisation de l'articulation, et
- l'amplitude entre la flexion et l'extension maximales de l'articulation comparativement à une articulation saine, compte tenu de la race du chien.

Les deux premiers paramètres appréciés par le vétérinaire tiennent compte de l'avis du propriétaire.

Les cinq paramètres sont appréciés par le vétérinaire selon le barème suivant :
- guérison, si tous les paramètres sont revenus à la normale,
- amélioration importante, si 3 paramètres au moins sont revenus à la normale,
- amélioration partielle, si 1 ou 2 paramètres sont revenus à la normale,
- échec, si aucun des 5 paramètres n'est revenu à la normale.

### 2. Résultats

### a) 30 jours après le début du traitement :

A la visite chez le vétérinaire, 30 jours après le début du traitement, on observe les résultats exprimés dans le tableau 2 ci-après :

**Tableau 2**

| | Echec | Amélioration partielle | Amélioration importante | Guérison |
|---|---|---|---|---|
| Formulation A | 35% | 50% | 15% | - |
| Formulation B | 55% | 40% | 5% | - |
| Formulation C | 55% | 45% | - | - |

Les propriétaires des chiens du groupe A ont signalé dans 13 cas sur 20 au vétérinaire que, dès le dixième jour de traitement, était apparue une amélioration générale de l'état de santé de leur animal, se traduisant par une mobilité spontanée de l'animal et une disparition de la douleur, souvent perçue lors de caresses.

On observe, après traitement par la composition A selon l'invention, une diminution significative du taux d'échecs et une augmentation significative du nombre d'animaux montrant une amélioration importante.

### b) 60 jours après le début du traitement :

A la visite chez le vétérinaire, 60 jours après le début du traitement, on observe les résultats exprimés dans le tableau 3 ci-après :

**Tableau 3**

| | Echec | Amélioration partielle | Amélioration importante | Guérison |
|---|---|---|---|---|
| Formulation A | 15% | 20% | 65% | - |
| Formulation B | 35% | 25% | 40% | - |
| Formulation C | 40% | 25% | 35% | - |

La formulation A selon l'invention apparaît nettement comme étant celle qui présente le meilleur effet thérapeutique, alors que les animaux traités ont reçu avec cette formulation une quantité de sulfate de chondroïtine moitié moindre de celle qui leur a été administrée avec les formulations B et C.

On observe, après traitement par la composition A selon l'invention, une diminution significative du taux d'échecs et une augmentation significative du nombre d'animaux montrant une amélioration importante.

## Revendications

1. Utilisation d'une préparation comprenant :
a) de 1 à 50% en poids, par rapport au poids total de la préparation, d'au moins un composé choisi parmi les sulfates de chondroïtine et leurs sels, et
b) de 1 à 66% en poids, par rapport au poids total de la préparation, d'au moins un composé choisi parmi le chitosan et ses sels, les dérivés de chitosan choisis par les N-acyl chitosans, les N-carboxyalkyl chitosans, les N-carboxyacyl chitosans, les O-carboxyalkyl chitosans, les déoxyglycit-1-yl chitosans, les hydroxyalkyl chitosans et leurs sels,
pour la réalisation d'une composition pour prévenir ou traiter les affections rhumatologiques par voie générale.

2. Utilisation selon la revendication 1, dans laquelle la préparation comprend un sulfate de chondroïtine sodique.

3. Utilisation selon la revendication 1 ou la revendication 2, dans laquelle la préparation comprend un composé choisi parmi le chitosan et ses sels d'acide adipique, d'acide ascorbique, d'acide formique, d'acide glycolique et d'acide lactique.

4. Utilisation selon l'une quelconque des revendications précédentes, dans laquelle le chitosan a un degré de désacétylation au moins égal à 80%.

5. Utilisation selon l'une quelconque des revendications précédentes, dans laquelle la préparation comprend en outre une quantité efficace d'un ou plusieurs inhibiteurs de radicaux libres.

6. Utilisation selon l'une quelconque des revendications précédentes, dans laquelle la composition est une composition phamiaceutique fonnulée pour être administrée par voie orale.

7. Utilisation selon l'une quelconque des revendications 1 à 5, dans laquelle la composition est un complément alimentaire.

8. Utilisation selon la revendication 6 ou la revendication 7, dans laquelle la composition comprend des quantités de sulfate de chondroïtine sodique et de chitosan adaptées pour apporter, par kg de poids vif et par jour, de à 25 mg de sulfate de chondroïtine sodique et de 5 à 50 mg de chitosan.

9. Utilisation selon la revendication 6 ou la revendication 7, dans laquelle la composition comprend des quantités de sulfate de chondroïtine sodique et d'un chitosan ayant un degré de N-désacétylation d'au moins 80%, permettant d'apporter, par kg de poids vif et par jour, de 10 à 20 mg et, de préférence, environ 15 mg de sulfate de chondroïtine et de 10 à 20 mg et, de préférence, environ 15 mg de chitosan.

10. Utilisation selon l'une quelconque des revendications précédentes, dans laquelle la composition est destinée à prévenir ou à traiter les arthroses.

## Claims

1. Use of a preparation comprising:
a) from 1 to 50% by weight, based on the total weight of the preparation, of at least one compound selected from chondroitin sulfates and their salts, and
b) from 1 to 66% by weight, based on the total weight of the preparation, of at least one compound selected from chitosan and its salts, chitosan derivatives selected from N-acyl chitosans, N-carboxyalkyl chitosans, N-carboxyacyl chitosans, O-carboxyalkyl chitosans, deoxyglycit-1-yl chitosans, hydroxyalkyl chitosans and their salts,
for producing a composition for the prevention or treatment of rheumatological complaints by general administration.

2. Use according to Claim 1 wherein the preparation comprises a sodium chondroitin sulfate.

3. Use according to Claim 1 or Claim 2 wherein the preparation comprises a compound selected from chitosan and its salts with adipic acid, ascorbic acid, formic acid, glycolic acid and lactic acid.

4. Use according to any one of the preceding claims wherein the chitosan has a degree of deacetylation of at least 80%.

5. Use according to any one of the preceding claims wherein the preparation also comprises an effective amount of one or more free radical inhibitors.

6. Use according to any one of the preceding claims wherein the composition is a pharmaceutical composition formulated for oral administration.

7. Use according to any one of Claims 1 to 5 wherein the composition is a food supplement.

8. Use according to Claim 6 or Claim 7 wherein the composition comprises sodium chondroitin sulfate and chitosan in amounts appropriate for the provision of 5 to 25 mg of sodium chondroitin sulfate and 5 to 50 mg of chitosan per kg of body weight per day.

9. Use according to Claim 6 or Claim 7 wherein the composition comprises sodium chondroitin sulfate and a chitosan having a degree of N-deacetylation of at least 80% in amounts appropriate for the provision of 10 to 20 mg, preferably about 15 mg, of chondroitin sulfate and 10 to 20 mg, preferably about 15 mg, of chitosan per kg of body weight per day.

10. Use according to any one of the preceding claims wherein the composition is intended for the prevention or treatment of arthroses.

## Patentansprüche

1. Verwendung einer Präparation, umfassend:
a) 1 bis 50 Gew.-%, bezogen auf das Gesamtgewicht der Präparation, wenigstens einer Verbindung, die unter Sulfaten von Chondroitin und ihren Salzen ausgewählt ist und
b) 1 bis 66 Gew.-%, bezogen auf das Gesamtgewicht der Präparation wenigstens einer Verbindung, die aus Chitosan und seinen Salzen, den Derivaten von Chitosan, ausgewählt unter N-Acylchitosanen, N-Carboxyalkylchitosanen, N-Carboxyalkylchitosanen, O-Carboxyalkychitosanen, Desoxyglycit-1-yl-chitosanen, Hydroxyalkylchitosanen und ihren Salzen, ausgewählt ist,
für die Herstellung einer Zusammensetzung zur Prävention oder Behandlung von rheumatischen Erkrankungen auf systemischem Weg.

2. Verwendung nach Anspruch 1, wobei die Präparation ein Natriumchondroitinsulfat umfasst.

3. Verwendung nach Anspruch 1 oder Anspruch 2, wobei die Präparation eine Verbindung, ausgewählt unter Chitosan und seinen Adipinsäure-, Ascorbinsäure-, Ameisensäure-, Glykolsäure- und Milchsäure-Salzen, umfasst.

4. Verwendung nach einem der vorangehenden Ansprüche, wobei das Chitosan einen Desacetylierungsgrad von wenigstens gleich 80% hat.

5. Verwendung nach einem der vorangehenden Ansprüche, wobei die Präparation außerdem eine wirksame Menge eines oder mehrerer Inhibitoren freier Radikale umfasst.

6. Verwendung nach einem der vorangehenden Ansprüche, wobei die Zusammensetzung eine pharmazeutische Zusammensetzung ist, die zur Verabreichung auf oralem Weg formuliert ist.

7. Verwendung nach einem der Ansprüche 1 bis 5, wobei die Zusammensetzung ein Nahrungsergänzungsmittel ist.

8. Verwendung nach Anspruch 6 oder Anspruch 7, wobei die Zusammensetzung Mengen an Natriumchondroitinsulfat und Chitosan umfasst, die so angepasst sind, dass sie pro kg Lebendgewicht und pro Tag 5 bis 25 mg Natriumchondroitinsulfat und 5 bis 50 mg Chitosan liefern.

9. Verwendung nach Anspruch 6 oder Anspruch 7, wobei die Zusammensetzung Mengen an Natriumchondroitinsulfat und einem Chitosan, das einen N-Desacetylierungsgrad von wenigstens 80% hat, umfasst, was es ermöglicht, pro kg Lebendgewicht und pro Tag 10 bis 20 mg und vorzugsweise etwa 15 mg Chondroitinsulfat und 10 bis 20 mg und vorzugsweise etwa 15 mg Chitosan zu liefern.

10. Verwendung nach einem der vorangehenden Ansprüche, wobei die Zusammensetzung zur Prävention oder Behandlung von Arthrosen bestimmt ist.
